Europäisches Patentamt

European Patent Office

Office européen des brevets

(19)

(11) Publication number: 0 337 733
A2

# EUROPEAN PATENT APPLICATION

(12)

(21) Application number: 89303570.9

(22) Date of filing: 11.04.89

(51) Int. Cl.⁴: **A 61 K 9/08**
**A 61 K 31/65**

(30) Priority: 13.04.88 US 181007

(43) Date of publication of application:
18.10.89  Bulletin  89/42

(84) Designated Contracting States:
AT BE CH DE FR GB IT LI LU NL SE

(71) Applicant: PFIZER INC.
235 East 42nd Street
New York, N.Y. 10017  (US)

(72) Inventor: Gibbs, David L.
250 Mercer Street
New York New York  (US)

(74) Representative: Bradbrook, Geoffrey William et al
PFIZER LIMITED Ramsgate Road
Sandwich Kent  (GB)

(54) Single dose intramuscular treatment of chlamydia trachomatis infections.

(57) A method of treating Chlamydia trachomatis infections in mammals, including humans, comprising administering intramuscularly, in a single dose, to a mammal requiring such treatment a composition comprising doxycycline or minocycline in a pharmaceutically acceptable vehicle that provides plasma concentrations of doxycycline or minocycline of at least about 0.1 micrograms per ml for a period of at least about 4-5 days.

EP 0 337 733 A2

**Description**

# SINGLE DOSE INTRAMUSCULAR TREATMENT OF CHLAMYDIA TRACHOMATIS INFECTIONS

The present invention relates to a single dose intramuscular treatment of Chlamydia trachomatis infections.

Chlamydia trachomatis is the pathogen responsible for most non-gonoccocal urethritis in males and cervicitis in females. Sexually transmitted chlamydia infections are presently treated for 7 to 10 days with oral tetracyclines, doxycycline or erythromycins. Unfortunately, many infections are asymptomatic and symptoms, when present, may disappear after only 48 hours of treatment. As a result, lack of patient compliance, which is frequent, results in further spread of disease.

Doxycycline (also referred to as alpha-6-deoxy-5-oxytetracycline) is described in United States Patent 3,200,149, assigned to Pfizer Inc. Doxycycline formulations useful in the method of the present invention are described in United States Patent 3,846,548 (the '548 patent), assigned to Pfizer Inc. Example I of the latter patent refers to administration of a single intramuscular dose of a doxycycline solution comprising Nikkol HCO-60 (a type of polyeoxyethylene hydrogenated castor oil) to dogs, rabbits and humans and shows blood levels at 24 hours. The human dose was 100 mg. The disclosure of the foregoing patents is hereby incorporated herein by reference.

I have found that the formulation referred to in the '548 patent, which has been sold only for intravenous use in Japan, and similar formulations may be administered as a single intramuscular dose that provides blood levels of at least about 0.05 micrograms per ml for a period of at least about 4-5 days. Such a single dose treatment is effective in treating Chlamydia trachomatis infections and substantially eliminates problems of patient compliance.

The present invention relates to a method of treating Chlamydia trachomatis infections in mammals, including humans, comprising administering intramuscularly, in a single dose, to a mammal requiring such treatment a composition comprising doxycycline or minocycline in a pharmaceutically acceptable vehicle that provides plasma concentrations of doxycycline of at least about 0.05 micrograms per ml, preferably at least about 0.1 micrograms per ml, for a period of at least about 4-5 days. As used herein and unless indicated otherwise, the term doxycycline includes both doxycycline and its pharmaceutically acceptable acid addition salts and the term minocycline includes both minocycline and its pharmaceutically acceptable acid addition salts.

For administration to the average adult human, the amount of doxycycline or minocycline in the foregoing composition is about 50 to about 150 mg, preferably about 100 mg. This amount of doxycycline may be provided by doxycycline base or by an amount of another form of doxycycline (i.e., a pharmaceutically acceptable acid addition salt of doxycycline such as the hydrochloride or the hyclate) that is equivalent to about 50 to about 150 mg of doxycycline base. Similarly, the required amount of minocycline may be provided by minocycline or by an amount of another form of minocycline (e.g., minocycline hydrochloride) that is equivalent to about 50 to about 150 mg of minocycline. For administration to other mammals or to humans weighing significantly more or less than average, the amount of doxycycline or minocycline is about 0.85 to about 2.6 mg per kilogram of body weight, preferably about 1.7 mg per kilogram of body weight. The expression "at least about 4-5 days" is intended to mean that in a typical patient population at least about half of the patients will have the specified minimum plasma concentrations for at least about 5 days and that substantially all of the patients will have the specified minimum plasma concentrations for at least about 4 days.

The injection used in the method of the present inventions preferably comprises, in a 5 ml aqueous injectable solution, (a) about 50 to about 150 mg of of doxycycline or minocycline; (b) about 450 to about 550 mg of polyoxyethylene hydrogenated castor oil; (c) a magnesium compound selected from the group consisting of magnesium chloride, magnesium ascorbate, magnesium lactate and magnesium gluconate, the molar ratio of magnesium compound to doxycycline or minocycline being about 1:1 to about 8:1; (d) an effective amount of an antioxidant; and (e) water. More preferably, the amount of a doxycycline or minocycline is about 100 mg.

Most preferably the injection comprises doxycycline hyclate or minocycline hydrochloride in an amount equivalent to about 100 mg of doxycycline or minocycline respectively, about 450 to about 550 mg of polyoxyethylene hydrogenated castor oil, about 90 to about 110 mg of magnesium chloride hexahydrate, about 18 to about 22 mg of thioglycerol, and about 4.0 to about 4.8 ml of water.

Preferably, the hydrochloride salt of doxycycline or minocycline and more preferably the hydrochloride salt of doxycycline is used in the method of the present invention. Most preferably, doxycycline hyclate is used. For a discussion of doxycycline and its salts and doxycycline formulations, see the Merck Index, Tenth Edition, Editor M. Windholz, Rahway N.J., pages 499 and 888 (1983) and the United States Pharmacopeia Twenty-First Revision, pages 358-361 (1984), the disclosures of which are hereby incorporated herein by reference. It should be understood that if any form of doxycycline or minocycline other than the free base is used, that form of doxycycline or minocycline should be present in an amount that is equivalent to about 50 to about 150 mg, preferably about 100 mg, of doxycycline or minocycline respectively.

Polyoxyethylene hydrogenated castor oils may be prepared by reacting hydrogenated castor oil with ethylene oxide. Preferably, about 40 to about 80 moles of ethylene oxide, more preferably, about 60 moles of ethylene oxide, is reacted with 1 mole of hydrogenated castor oil to prepare the polyoxyethylene hydrogenated castor oil for use in the method of the present invention.

Polyoxyethylene hydrogenated castor oils that may be used in the method of the present invention are available from Nikkol Chemicals Co., Ltd, Tokyo, Japan. Such materials include Nikkol HCO-40, Nikkol HCO-50, Nikkol HCO-60 and Nikkol HCO-80. The CTFA adopted names for the materials sold as Nikkol HCO-40, Nikkol HCO-50 and Nikkol HCO-60 are PEG-40 hydrogenated castor oil, PEG-50 hydrogenated castor oil and PEG-60 hydrogenated castor oil, respectively. The preferred polyoxyethylene hydrogenated castor oil is Nikkol HCO-60. For a discussion of polyoxyethylene hydrogenated castor oils, see pages 221-224 of the Handbook of Pharmaceutical Excipients (American Pharmaceutical Association, Washington, D.C. 1986), the disclosure of which is hereby incorporated herein by reference. Oils that are pharmaceutically acceptable and well tolerated may be substituted for polyoxyethylene hydrogenated castor oils to provide a suitable long acting formulation. If such an oil does not also function as a surfactant (as do polyoxyethylene hydrogenated castor oils), it may be necessary to add a pharmaceutically acceptable surfactant that is effective to solubilize the doxycycline or the minocycline that is used.

Magnesium ions react with doxycycline and minocycline to form, respectively, magnesium-doxycyline and magnesium-minocycline chelates. Suitable sources of magnesium ions include magnesium chloride, ascorbate, magnesium lactate, and magnesium gluconate. The molar ratio of magnesium to doxycycline in these compositions is one that is in the range of from about 1:1 to about 8:1 with the preferred ratio being from about 1:1 to about 4:1. The preferred source of magnesium ions is magnesium chloride, more preferably magnesium chloride hexahydrate. When magnesium chloride hexahydrate is used as the magnesium compound, the aforementioned 5 ml aqueous injectable solution preferably contains about 90 to about 110 mg of magnesium chloride hexahydrate.

In order to ensure the color and potency stabilities of the injectable solutions prepared in accordance with this invention, a suitable antioxidant is preferably added, such as sodium or magnesium formaldehyde sulfoxylate (about 0.2 to about 0.5 percent w/v); sodium sulfite, metabisulfite or bisulfite (about 0.1 to about 0.2 percent w/v); sodium sulfide (about 0.002 to about 0.004 percent w/v; alpha-monothioglycerol (also referred to as thioglycerol) (about 0.4 to about 1.0 percent w/v); or thiosorbitol (about 0.4 to about 1.0 percent w/v). The preferred antioxidant is thioglycerol. When thioglycerol is used as the antioxidant, the aforementioned 5 ml aqueous injectable solution preferably contains about 18 to about 22 mg of thioglycerol.

The pH of the aqueous doxycycline or minocycline compositions are adjusted to between 5.0 and 7.0 until a clear solution is obtained. Depending on the nature of the final pharmaceutical composition, the pH may be adjusted with a mineral acid such as hydrochloric acid or an organic acid such as citric acid or lactic acid. For basic pH adjustment, suitable inorganic bases include ammonium or sodium hydroxide and organic bases such as aminomethane, dimethylaminomethanol, diethylaminoethanol, dimethylamine, diethylamine, trimethylamine, triethylamine, and preferably 2-aminoethanol.

The single dose intramuscular injection described herein may be administered to a patient at a single injection site or the dose may be divided and administered at 2 or more injection sites. In addition to its usefulness in treating sexually transmitted infections caused by Chlamydia trachomatis, the single dose intramuscular injection may also be used in treating trachoma infections which are caused by Chlamydia trachomatis.

In order to reduce pain at the injection site, a local anesthetic such as lidocaine hydrochloride may be added to the injectable formulation. Generally, however, such an anesthetic is not necessary.

The following Example illustrates the preparation of a composition that is useful in the method of the present invention.

## Example 1

The following ingredients are combined under a nitrogen atmosphere as described below to prepare a batch of 200 liters providing a dose equivalent to 20 mg of doxycycline per ml:

| Ingredient | mg/5ml | kg/batch |
|---|---|---|
| Doxycycline hyclate | 127.9 * | 5.116 |
| Magnesium chloride, hexahydrate | 101.6 | 4.064 |
| Nikkol HCO-60 | 500.0 | 20.000 |
| Monothioglycerol | 20.0 | 0.800 |
| Monoethanolamine 99% | Approx. 30 | 1.2 |
| Water for injection | Approx. 4.370 | 174.8 |
| Nitrogen | As required | As required |

* Includes a 10% overage

Heat 164.8 kg of the water for injection to 70-80°C. In a separate vessel, melt 20 ko. of Nikkol HCO-60 at 70-80°C. Dissolve the melted Nikkol HCO-60 in the heated water for injection, with agitation, and then cool the solution to room temperature. Add 4.064 kg of the magnesium chloride hexahydrate to the remaining 10 kg of water and dissolve the added material with stirring. Add the resulting solution to the Nikkol HCO-60 solution and stir until homogeneous. Then add and dissolve 5.116 kg of the doxycycline hyclate. To the resulting solution, add very slowly, with agitation, the monoethanolamine and adjust the pH to between 5.0 to 5.3. After each addition of monoethanolamine, stir until a clear solution is obtained and do not allow the temperature to exceed 25°C. To the resulting solution, add 0.8 kg of the monothioglycerol. Then measure the pH and, if necessary, readjust the pH. Allow the resulting solution to stand overnight for approximately 16 hours to permit the solution to reach pH equilibrium. Then measure the pH and, if necessary, readjust the pH. Then filter the solution through a sterile filter (0.22 um pore size) preceded by prefilter (0.45 um pore size) and then aseptically fill and seal the filtered solution into filtered nitrogen purged 5 ml clear amber glass ampules.

The doxycycline solution is light sensitive and, during manufacturing and filling, the product should be protected against light. Also, the solution or its components should not come in contact with metals such as iron, copper, and zinc, which may bring about discoloration or darkening of the end product.

### Example 2

A formulation prepared by the method of Example 1 was administered intramuscularly to 14 subjects. The foregoing dose produced plasma concentrations equal to or above 0.1 micrograms per ml for 96 hours in all subjects and for 120 hours in 7 of the 14 subjects.

## Claims

1. Use of doxycycline or minocycline for the manufacture of a medicament for administration intramuscularly, in a single dose, in the treatment of Chlamydia trachomatis infections in mammals, said medicament comprising from about 0.85 to about 2.6 mg of doxycycline or minocycline per kilogram of body weight in a pharmaceutically acceptable vehicle that provides plasma concentrations of doxycycline or minocycline of at least about 0.05 micrograms per ml for at least about 4-5 days.

2. Use according to Claim 1, wherein said composition comprises about 1.7 mg of doxycycline or minocycline per kilogram of body weight.

3. Use of doxycycline or minocycline for the manufacture of a medicament for administration intramuscularly, in a single dose, in the treatment of Chlamydia trachomatis infections in humans, said medicament comprising from about 50 to about 150 mg of doxycycline or minocycline in a pharmaceutically acceptable vehicle that provides plasma concentrations of doxycycline or minocycline of at least about 0.05 micrograms per ml for at least about 4-5 days.

4. Use according to any one of Claims 1 to 3, wherein said plasma concentrations are at least about 0.1 micrograms per ml for at least about 4-5 days.

5. Use according to any preceding claim, wherein said vehicle comprises polyoxyethylene hydrogenated castor oil.

6. Use according to Claim 5, wherein said polyoxyethylene hydrogenated castor oil is prepared by reacting about 1 mole of hydrogenated castor oil with about 60 moles of ethylene oxide.

7. Use according to any preceding claim, wherein said doxycycline is in the form of its hydrochloride salt.

8. Use according to any preceding claim, wherein said medicament comprises, in a 5 ml aqueous injectable solution, (a) from about 50 to about 150 mg of doxycycline or minocycline; (b) from about 450 to about 550 mg of polyoxyethylene hydrogenated castor oil; (c) a magnesium compound selected from magnesium chloride, magnesium ascorbate, magnesium lactate and magnesium gluconate, the molar ratio of magnesium compound to doxycycline or minocycline being from about 1:1 to about 8:1; (d) an effective amount of an antioxidant; and (e) water.

9. Use according to any preceding claim, wherein said medicament comprises about 100 mg of doxycycline or minocycline.

10. Use according to Claim 8 or 9, wherein said medicament comprises about 100 mg of doxycycline hyclate or minocycline hydrochloride, about 450 to about 550 mg of polyoxyethylene hydrogenated castor oil, about 90 to about 110 mg of magnesium chloride hexahydrate, about 18 to about 22 mg of thioglycerol, and about 4.0 to about 4.8 ml of water.